# EUROPÄISCHE PATENTANMELDUNG

(11) **EP 3 195 854 A1**
(43) Veröffentlichungstag der Anmeldung: **26.07.2017**
(21) Anmeldenummer: 16152391.5
(22) Anmeldetag: 22.01.2016
(51) Int. Cl.: A61K 8/49, A61Q 19/08

(54) **KOSMETISCHE BEHANDLUNG GESUNDER HAUT, INSBESONDERE GEALTERTER HAUT**

(71) Anmelder: Tomorrowlabs GmbH, 1010 Wien (AT)
(72) Erfinder: Thor, Dominik., 1010 Wien (AT); Duscher, Dominik., 4020 Linz (AT)
(74) Vertreter: Sonn & Partner Patentanwälte

(57) **Zusammenfassung**

Die vorliegende Erfindung betrifft die Verwendung von Deferipron zur kosmetischen Behandlung einer gesunden Hautstelle eines Individuums, umfassend die Anwendung einer Deferipron enthaltenden Zusammensetzung auf die Hautstelle; insbesondere gegen Zeichen der Hautalterung ("Anti-Aging"), und zur Erhöhung der Elastizität und Straffheit der Haut. Ferner wird eine Zusammensetzung offenbart, welche zu dieser Verwendung geeignet ist, umfassend Deferipron in einer Konzentration (w/w) von 1%-10%.

## Beschreibung

Das Gebiet der Erfindung ist das der kosmetischen Behandlungen gesunder Haut, insbesondere gegen Zeichen der Hautalterung ("Anti-Aging") bzw. zur Erhöhung der Elastizität und/oder Straffheit der Haut.

Mit fortschreitendem Alter erfolgt beim Menschen ein schrittweiser Verlust der Straffheit (Festigkeit) und Elastizität der Haut, was, zusammen mit der Verringerung und der Veränderung des Volumens an Fett, Muskeln, Knochengewebe, zu Gesichtsfalten und einem älter aussehenden Gesicht führt. Als Folge entstehen im Laufe der Zeit feine Fältchen und Falten. Im Gesicht gehören anfänglich nur vorübergehende Gesichtslinien - insbesondere feine Fältchen, Nasolabialfalten, Marionettenfalten, Lippenfältchen, Stirnfalten, Krähenfüße und Glabellafalten - zu den Zeichen der Hautalterung. Man unterscheidet zwischen chronologischer und extrinsischer Hautalterung.

Chronologische (intrinsische) Hautalterung wird durch endogene, genetisch determinierte Faktoren ausgelöst. Sowohl in Dermis als auch Epidermis kommt es alterungsbedingt zu unerwünschen Veränderungen. Insbesondere kommt es zu Verlust der Elastizität und der Straffheit der Haut, was sich in Altersanzeichen wie Falten, Fältchen, schrumpeliger Haut und schlaffer Haut niederschlagen kann.

Zusätzlich können schädliche exogene Faktoren, beispielsweise UV-Licht und/oder chemische Noxen, kumulativ wirksam werden und beispielsweise den endogenen Alterungsprozess auf negative Weise ergänzen oder beschleunigen. Hierbei spricht man von extrinsischer Hautalterung. Auch die Art der Lebensführung (z.B. in Bezug auf Rauchen, ungesunde Ernährung und Alkoholkonsum) kann den Einfluss dieser exogenenen Faktoren erhöhen und dadurch die Hautalterung beschleunigen.

Im Stand der Technik sind Präparate zur Verminderung der Zeichen der Hautalterung, oft auch als Anti-Aging-Präparate bezeichnet, bekannt. Beispielsweise offenbart die EP 0 945 126 A2 eine kosmetische Zusammensetzung bestehend aus Carnitin und/oder einem Acylcarnitin, und mindestens einem Antioxidans, zum genannten Zweck.

Übrigens ist festzuhalten, dass Hautalterung (bzw. als unästhetisch empfundene Haut im Allgemeinen) üblicherweise keine Krankheit darstellt, sondern durch physiologische Prozesse verursacht wird - somit ist die Haut trotz der genannten Altersanzeichen im Allgemeinen als gesund zu betrachten.

Trotz der zahlreichen im Stand der Technik vor allem unter dem Schlagwort "Anti-Aging" beworbenen kosmetischen Präparate besteht weiterhin, aufgrund der oft unzureichenden Wirksamkeit und auch aufgrund der immer höheren Lebenserwartung und dem steigenden allgemeinem Wunsch nach jugendlichem Aussehen in vielen Kulturen, Bedarf an neuen kosmetischen Wirkstoffen mit verbesserter Wirkung, z.B. was die Verbesserung des Hautbildes und insbesondere Anti-Aging betrifft.

Daher besteht eine Aufgabe der vorliegenden Erfindung darin, einen effektiven Wirkstoff zur kosmetischen Behandlung einer gesunden Hautstelle eines Individuums, insbesondere zu Anti-Aging-Zwecken, zur Verfügung zu stellen.

Überraschenderweise hat sich im Zuge der vorliegenden Erfindung Deferipron (3-Hydroxy-1,2-dimethylpyridin-4-on, FERRIPROX®) als effektiver kosmetischer Wirkstoff, unter anderem zur Verbesserung bzw. Glättung des Hautbildes, und insbesondere zu Zwecken des Anti-Aging, herausgestellt.

Die vorliegende Erfindung betrifft folglich die Verwendung von Deferipron zur kosmetischen Behandlung einer gesunden Hautstelle eines Individuums. Die Verwendung umfasst die Anwendung einer Deferipron enthaltenden Zusammensetzung auf die Hautstelle.

Bei den angestellten experimentellen Studien im Zuge der vorliegenden Erfindung hat sich unerwartet gezeigt, dass Deferipron bei Probanden zu einem besseren Hautbild führt, die Elastizität und Straffheit der Haut erhöht und Falten bzw. Fältchen verringert (vgl. Beispiel 1).

Wie sich aus den im Folgenden beschriebenen Dokumenten ergibt, sind zwar im Stand der Technik einige Anwendungen von Deferipron auf der Haut bekannt, doch diese beziehen sich auf die Vorbeugung oder Behandlung von pathologischen Störungen, oder Krankheiten, und nicht auf eine kosmetische Behandlung von gesunder Haut (die sich jedoch in einem kosmetisch unerwünschten Zustand befindet):

So bezieht sich die WO 2004/045609 A2 auf Hydroxypyridone zur lokalen Behandlung von Störungen der Mikrozirkulation der Haut wie Rosacea und Purpura actinica.

Die WO 01/17497 A1 offenbart 3-Hydroxypyr(id)on-Derivate zur Behandlung von Pigmentierungsstörungen wie Melasma.

Die WO 2008/035152 A1 betrifft ein Verfahren zur Entfernen von Augenringen und hat großteils Durchgriffsansprüche zum Gegenstand. Im Dokument werden Augenringe im wesentlichen als Symptom einer pathologischen Störung dargestellt, hauptsächlich verursacht durch undichte Kapillaren ("mainly driven by leaky capillaries"), wobei die Brüchigkeit der kleinen Blutgefäße ("micro-vessel fragility") erhöht ist.

Das US Patent Nr. 5,487,884 bezieht sich auf photoprotektive Zusammensetzungen mit Chelator zur Verhinderung von Hautschäden.

Die US 2013/0109664 A1 betrifft Pflegeprodukte mit einem Pyrithion und einem Eisenchelator gegen Pilzbefall.

Die WO 2006/084210 A2 bezieht sich auf ein Verfahren zum Behandeln von Individuen, die unter einer Infektion oder Virulenz von Pathogenen leiden.

Jene Dokumente nehmen die vorliegende Erfindung nicht vorweg und führen auch nicht zu ihr hin; sie versuchen nicht einmal, die erfindungsgemäße Aufgabe zu lösen.

Da sich Deferipron im Zuge der vorliegenden Erfindung als hervorragender Anti-Aging-Wirkstoff herausgestellt hat (vgl. Beispiel 1), besteht in einer bevorzugten Ausführungsform der vorliegenden Erfindung die genannte gesunde Hautstelle aus gealterter Haut, insbesondere aus chronologisch gealterter Haut. Bevorzugt weist die Hautstelle ein oder mehrere Merkmale für gealterte Haut auf, ausgewählt aus Falten, Fältchen, schrumpeliger Haut, schlaffer Haut, Verminderung der Elastizität im Vergleich zum Durchschnitt einer Kontrollgruppe mit Durchschnittsalter von etwa 20 Jahren, 25 Jahren oder 30 Jahren, und Verminderung der Straffheit im Vergleich zum Durchschnitt einer Kontrollgruppe mit Durchschnittsalter von etwa 20 Jahren, 25 Jahren oder 30 Jahren; insbesondere ausgewählt aus feine Fältchen (insbesondere unter den Augen), Nasolabialfalten, Marionettenfalten, Lippenfältchen, Stirnfalten, Krähenfüße und Glabellafalten. Vorzugsweise ist die gealterte Haut keine verheilende Wunde bzw. keine kürzlich (z.B. in den letzten 10, 20, 30, 100, 300, 1000, 5000 oder 10000 Tagen) aus Wundheilung hervorgegangene Haut.

Insbesondere haben Studien im Zuge der vorliegenden Erfindung ergeben, dass Deferipron bei Probanden die Elastizität bzw. Straffheit der Haut erhöht hat (vgl. Beispiel 1). Daher ist in einer bevorzugten Ausführungsform die erfindungsgemäße Verwendung zur Erhöhung der Elastizität und/oder Straffheit der Hautstelle vorgesehen. Die Straffheit wird auch als Festigkeit ("firmness") bezeichnet.

Die Elastizität einer Hautstelle kann bestimmt werden, indem untersucht wird, in welcher Zeit die Hautstelle nach einer durch Krafteinwirkung verursachten Verformung wieder in ihre Ursprungsform zurückkehrt. Demgegenüber kann die Straffheit einer Hautstelle bestimmt werden, indem untersucht wird, welcher Kraftaufwand erforderlich ist, um eine bestimmte Verformung der Hautstelle überhaupt zu bewirken. Probanden können insbesondere die erste Untersuchung selbst an sich durchführen und z.B. in einem Fragebogen darüber Auskunft geben (vgl. Beispiel 1).

Weitere Quantifizierungen von Elastizität bzw. Straffheit der Hautstelle sind beispielsweise mit technischen Vorrichtungen wie dem CUTOMETER® (Courage + Khazaka electronic GmbH) möglich. Vorteilhafterweise ist die erfindungsgemäße Verwendung zur Verbesserung eines oder mehrerer Hautparameter, ausgewählt aus den R-Parametern R0-R9, insbesondere R0 (repräsentiert die Kraft, sich dem Ansaugen zu widersetzen; also die Festigkeit bzw. Straffheit), R2 (Bruttoelastizität), R5 (Nettoelastizität), R7 (Anteil der Elastitizät an der Gesamtkurve) und R9 (Ermüdung der Haut/Fatigue), den F-Parametern F1-F4 und den Q-Parametern Q0-Q3. Anwendungsbeispiele finden sich in der Literatur, unter anderem in Ryu, Hyo Sub, et al. "Influence of age and regional differences on skin elasticity as measured by the Cutometer®." Skin Research and Technology 14.3 (2008): 354-358.

Für solche Quantifizierungen geeignete technische Vorrichtungen bzw. Verfahren sind unter anderem auch in den folgenden Schriften offenbart: EP 0 362 616 A1, EP 2 301 436 A1, WO 2009/144680 A1 und WO 2003/105689 A1. Insbesondere sind in diesen Schriften genannte Elastizitäts/Straffheits-Parameter zur Bestimmung der Erhöhung von Hautelastizität bzw. Hautstraffheit geeignet.

Die vorteilhafte Wirkung von Deferipron schlägt sich strukturell insbesondere in der Dermis, vor allem im Stratum reticulare, nieder. Insbesondere wird die Erhöhung der Elastizität bzw. Straffheit hier bewirkt. Folglich ist in einer bevorzugten Ausführungsform die erfindungsgemäße Verwendung zur Behandlung der Dermis der Hautstelle, insbesondere des Stratum reticulare, vorgesehen. Insbesondere soll eine strukturelle Änderung der Dermis der Hautstelle, insbesondere des Stratum reticulare, bewirkt werden bzw. wird sie bewirkt.

Vorteilhafterweise ist die erfindungsgemäße Verwendung zum Zwecke der Behandlung eines Zeichens der Hautalterung, vorzugsweise in der Dermis der Hautstelle, vorgesehen. Bevorzugt ist besagte Hautalterung die chronologische Hautalterung. Die Zeichen der Hautalterung sind bevorzugt ausgewählt aus aus Falten, Fältchen, schrumpeliger Haut und schlaffer Haut. Insbesondere sind die Zeichen der Hautalterung ausgewählt aus feinen Fältchen (insbesondere unter den Augen), Nasolabialfalten, Marionettenfalten, Lippenfältchen, Stirnfalten, Krähenfüße und Glabellafalten.

Da sich die Hautalterung naturgemäß mit steigendem Alter des Individuums immer stärker bemerkbar macht, beträgt das Alter des mit Deferipron behandelten menschlichen Individuums in einer bevorzugten Ausführungsform zumindest 30 Jahre, bevorzugt zumindest 40 Jahre, mehr bevorzugt zumindest 50 Jahre, noch mehr bevorzugt zumindest 60 Jahre, insbesondere zumindest 70 Jahre oder gar 80 Jahre.

Die Hautstelle, auf der Deferipron angewandt wird, befindet sich bevorzugt im Gesicht, gegebenenfalls mit Ausnahme der Augenpartien oder der unteren Augenpartien (u.a. um Augenreizungen besser zu vermeiden), am Hals, am Rumpf oder an den Gliedmaßen des Individuums.

Ein weiterer Aspekt der vorliegenden Erfindung betrifft eine Zusammensetzung, die zur erfindungsgemäßen Verwendung geeignet bzw. vorgesehen ist. Diese Zusammensetzung umfasst Deferipron in einer kosmetisch wirksamen Konzentration, d.h. in einer Konzentration, bei der, abgestimmt auf die jeweilige Verabreichungsform, eine kosmetisch (bevorzugt in der Dermis, insbesondere im Stratum reticulare) wirksame Dosis von Deferipron an das Individuum bei üblicher Anwendung (z.B. Einschmieren der Hautstelle, 1-3x täglich) verabreicht werden kann. Zweckmäßigerweise liegt Deferipron in einer Konzentration (w/w) von 1%-10%, bevorzugt von 2%-8%, mehr bevorzugt von 3%-7%, noch mehr bevorzugt von 4%-6%, insbesondere von im Wesentlichen 5%, vor.

Zweckmäßigerweise ist die erfindungsgemäße Zusammensetzung zur täglichen Anwendung auf der Hautstelle (bevorzugt 1x, 2x oder 3x täglich) vorgesehen, insbesondere über einen längeren Zeitraum wie über zumindest eine Woche, bevorzugt über zumindest zwei Wochen, noch mehr bevorzugt über zumindest einen Monat, insbesondere über zumindest ein Jahr oder gar über zumindest fünf Jahre.

Damit Deferipron seine Wirkung in der Dermis noch besser entfalten kann, umfasst die Zusammensetzung in einer bevorzugten Ausführungsform ferner ein kosmetisch verträgliches Mittel zur Verstärkung der Hautpenetration von Deferipron. Geeignete Penetrationsverstärker sind beispielsweise ausgewählt aus der Gruppe bestehend aus C12-C15 Alkylbenzoat, Pentylenglycol, Polyethylenglycol, Ethoxydiglycol, Dimethylsulfoxid, Natriumlaurylsulfat, Polysorbatpolyethylensorbitan-monolaurat, Lecithin und Gemischen davon.

Die Zusammensetzung kann ferner weitere kosmetisch geeignete Inhaltsstoffe beinhalten, vorzugsweise ausgewählt aus Hyalorunansäure, Vitaminen (z.B. Vitamin C, Vitamin E, Vitamin A, insbesondere all-trans-Retinylpalmitat), Antioxidantien (z.B. alpha-Hydroxycarbonsäuren wie Salicylsäure, Gluconsäure, Glycolsäure, Apfelsäure), Imidazol, alpha-Tocopherol, Eisenkomplexbildnern (z.B. EDTA, alpha-Hydroxyfettsäuren), Coenzymen (beispielsweise Ubichinon (Q-10), Pantothensäure, Panthenol, Liponsäure, Niacin), Hilfsfaktoren (z.B. L-Carnitin, Uridin), Stoffwechselsubstraten (z.B. Hexosen, Pentosen, Fettsäuren), zelluläre Energieüberträgern (wie Riboflavin, Guanosin, Kreatin, Guanin, Adenin, Adenosin, Nicotin, Nicotinamid), intermediären Stoffwechselprodukten (z.B. Zitronensäure, Pyruvat) und Glutathion, insbesondere Vitamin A, Vitamin C und/oder Hyalorunansäure. Ausgewählte dieser Inhaltsstoffe können zweckmäßigerweise in Konzentrationen von 0,001% (oder 0,01% oder 0,1% oder 1%) bis 5% oder 10% oder 12% (w/w) vorliegen.

In einer besonders bevorzugten Ausführungsform sind in der Zusammensetzung als kosmetische Wirkstoffe neben Deferipron im Wesentlichen nur Vitamin A, Vitamin C und/oder Hyalorunansäure enthalten. Insbesondere umfasst die Zusammensetzung auch ein kosmetisch verträgliches Mittel zur Verstärkung der Hautpenetration von Vitamin A, Vitamin C und/oder Hyalorunansäure. Geeignete Penetrationsverstärker sind beispielsweise ausgewählt aus der Gruppe bestehend aus C12-C15 Alkylbenzoat, Pentylenglycol, Polyethylenglycol, Ethoxydiglycol, Dimethylsulfoxid, Natriumlaurylsulfat, Polysorbatpolyethylensorbitan-monolaurat, Lecithin und Gemischen davon.

Die erfindungsgemäße Zusammensetzung kann in jeglicher kosmetischen Verabreichungsform, die zur Anwendung auf der Haut geeignet ist, vorgesehen sein. Gängige kosmetische Verabreichungsformen sind unter anderem Cremes (W/O, O/W, W/OW), Gele, Lotionen und Milchen.

In der erfindungsgemäßen Zusammensetzung sind üblicherweise ein oder mehrere kosmetisch verträgliche Träger und gegebenenfalls Hilfsstoffe enthalten. Beispielsweise sind diese ausgewählt aus Ölen, Fetten, Wachsen, Emulgatoren, ein- und mehrwertigen Alkoholen, Duftstoffen, Farbstoffe, Konservierungsmitteln, Verdickungsmitteln, Puffersubstanzen, Trübungsmitteln, anionische, kationischen, ampholytischen, zwitterionischen und/oder nichtionogenen Tensiden sowie Wasser, und anderen dem Fachmann vertrauten kosmetischen Inhaltsstoffen.

Weitere Definitionen:

Unter "gesunder" Haut (bzw. Hautstelle) wird Haut (bzw. eine Hautstelle) verstanden, die von keiner üblicherweise behandlungsbedürftigen Krankheit oder pathologischen Störung (z.B. Infektionen, bullöse Dermatosen, papulosquamöse Hautkrankheiten, Urtikaria, Erythem, Dermatitis solaris acuta, Störungen der Mikrozirkulation der Haut wie Rosacea und Purpura actinica, und Pigmentierungsstörungen wie Melasma, von üblicherweise behandlungsbedürftigem Schweregrad) betroffen ist, vorzugsweise von keiner Krankheit oder pathologischen Störung (z.B. Infektionen, bullöse Dermatosen, papulosquamöse Hautkrankheiten, Urtikaria, Erythem, Dermatitis solaris acuta, Störungen der Mikrozirkulation der Haut wie Rosacea und Purpura actinica, und Pigmentierungsstörungen wie Melasma) betroffen ist. Bevorzugt ist das Individuum auch keine Risikoperson (z.B. mit einem Inzidenzrisiko von über 10%, 20%, 30%, 40%, 50%, 60% oder 70% über die nach dem Zeitpunkt der geplanten Anwendung verbleibende Lebenszeit) oder Hochrisikoperson (z.B. mit einem Inzidenzrisiko von über 80%, 90%, oder 95% über die nach dem Zeitpunkt der geplanten Anwendung verbleibende Lebenszeit) für die Ausbildung einer oder mehrerer der genannten Krankheiten. Der mit der üblichen Hautalterung in Verbindung stehende Verlust von Elastizität und/oder Straffheit in der Haut bzw. die Faltenbildung in der Haut (und andere hierin erwähnte Zeichen der Hautalterung) ist selbstverständlich nicht als Krankheit bzw. pathologische Störung zu betrachten. Gesunde Haut ist bevorzugt unverletzt, insbesondere auch unbeschädigt, ferner weist sie bevorzugt keine Einblutungen und/oder Hämatome auf, ferner ist sie bevorzugt dermatologisch unauffällig. Gesunde Haut kann auch als "physiologisch normale Haut" bezeichnet werden.

Unter einer "kosmetischen Behandlung" wird hierin das In-Berührung-Bringen eines kosmetischen Wirkstoffs (z.B. Deferipron) mit einem Individuum verstanden, bevorzugt durch eine Anwendung auf der Haut des Individuums (z.B. Einschmieren). Insbesondere hat eine "kosmetische Behandlung" (einer Hautstelle), z.B. im Gegensatz zu einer Vorbeugung, (gegebenenfalls nur im Wesentlichen) die Abmilderung und/oder Behebung eines kosmetisch unerwünschten Zustandes (auf der Hautstelle), welcher jedoch schon (auf der Hautstelle) vorhanden ist, zum Zweck. Unter einem "kosmetisch unerwünschten Zustand" wird insbesondere ein hierin genannter kosmetisch unerwünschter Zustand verstanden, z.B. ausgewählt aus gealterter Haut, dem Vorhandsein eines Zeichens der Hautalterung wie Falten, Fältchen, schrumpeliger Haut oder schlaffer Haut, Verminderung an Straffheit und/oder Elastizität (im Vergleich zum Durchschnitt einer Kontrollgruppe wie hierin ausgeführt), usw. Eine "kosmetische Behandlung" ist vorzugsweise nicht therapeutisch, d.h. sie zielt nicht auf eine Behandlung oder Vorbeugung einer Krankheit oder pathologischen Störung ab.

Unter einem kosmetischen Wirkstoff wird hierin ein Stoff verstanden, der in der Haut, bevorzugt in der Dermis, insbesondere im Stratum reticulare direkt wirksam sein soll bzw. ist, d.h. einen direkten kosmetischen Effekt auf die Haut, bevorzugt auf die Dermis, insbesondere das Stratum reticulare, haben soll bzw. hat. Demnach ist beispielsweise ein UV-Filter wie er in Sonnenschutzmitteln gebräuchlich ist kein kosmetischer Wirkstoff. Beispielsweise versteht der Fachmann auch Stoffe, welche einer kosmetischen Zusammensetzung im Wesentlichen nur beigefügt sind, um z.B. die Lagerstabilität, das Aussehen, den Geruch, den Geschmack etc. der kosmetischen Zusammensetzung zu verbessern, nicht als kosmetische Wirkstoffe, sondern als Hilfsstoffe.

Die vorliegende Erfindung wird ferner durch die folgenden Figuren und Beispiele illustriert, auf die sie selbstverständlich nicht eingeschränkt ist.

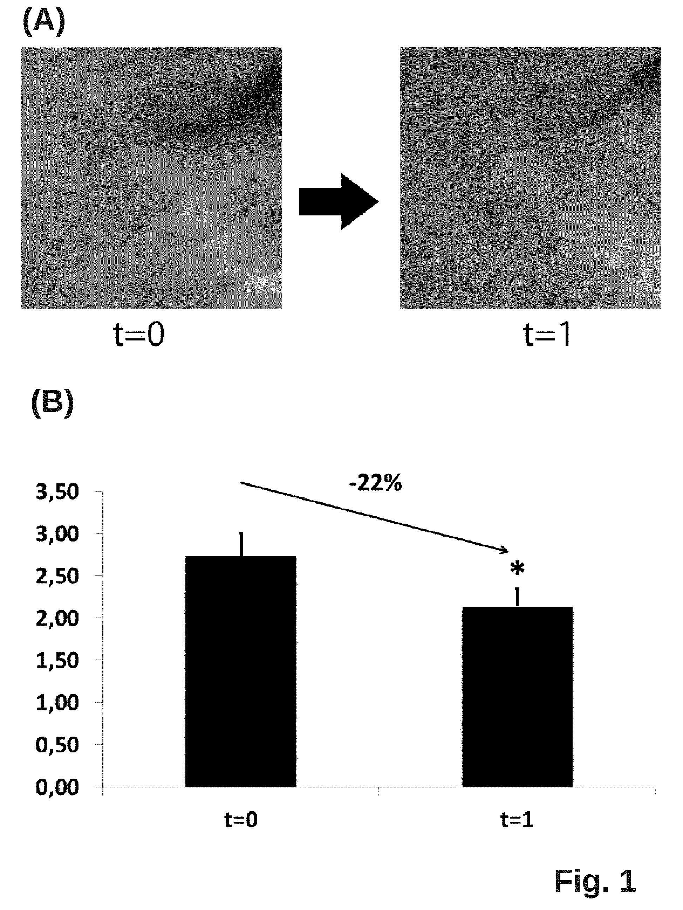

Figur **1** - Vergleich von Krähenfüßen vor der Behandlung (t=0) und nach der Behandlung **(t=1).** (A) Fotografierter Gesichtsbereich eines typischen Probanden bei Behandlungsbeginn (t=0) und nach der Behandlung (t=1). Das Ausmaß der Krähenfüße wurde durch die Behandlung mit Deferipron reduziert. (B) Median der Expertenbewertung der Krähenfüße bei Behandlungsbeginn (t=0) und nach Ende der Behandlung (t=1) anhand einer fünfstufigen Skala von 1-5. Es zeigte sich eine statistisch signifikante Verbesserung durch die Behandlung mit Deferipron. n=20 (20 Probanden, die von 7 Experten bewertet wurden), die kleinen Balken repräsentieren die jeweilige Standardabweichung.

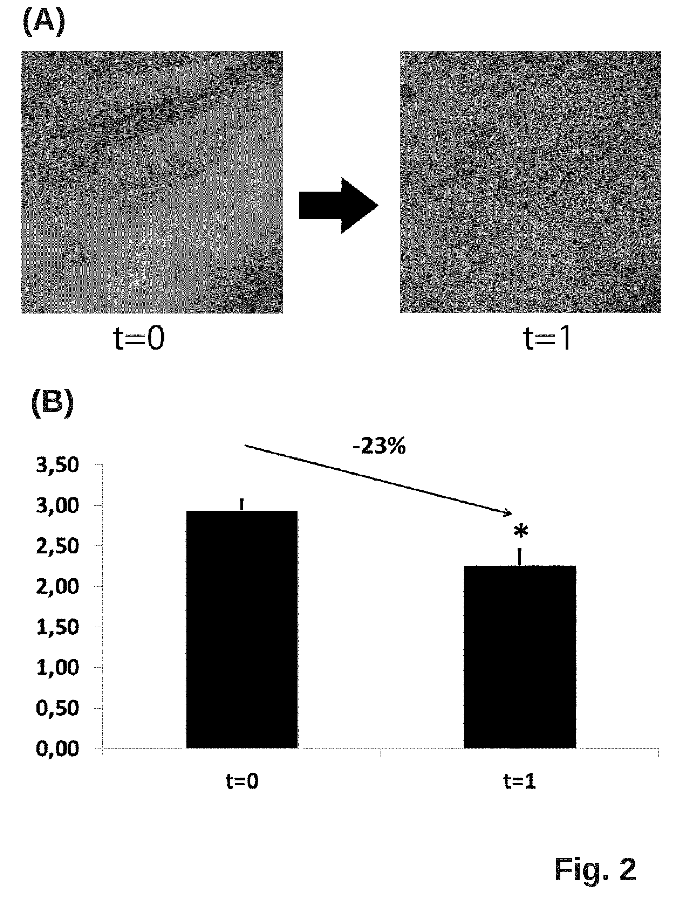

**Figur 2** **- Vergleich von feinen Fältchen vor der Behandlung und nach der Behandlung.** (A) Fotografierter Gesichtsbereich eines typischen Probanden bei Behandlungsbeginn (t=0) und nach der Behandlung (t=1). Das Ausmaß der feinen Fältchen wurde durch die Behandlung mit Deferipron reduziert. (B) Median der Expertenbewertung der feinen Fältchen bei Behandlungsbeginn (t=0) und nach Ende der Behandlung (t=1) anhand einer fünfstufigen Skala von 1-5. Es zeigte sich eine statistisch signifikante Verbesserung durch die Behandlung mit Deferipron. n=20 (20 Probanden, die von 7 Experten bewertet wurden), die kleinen Balken repräsentieren die jeweilige Standardabweichung.

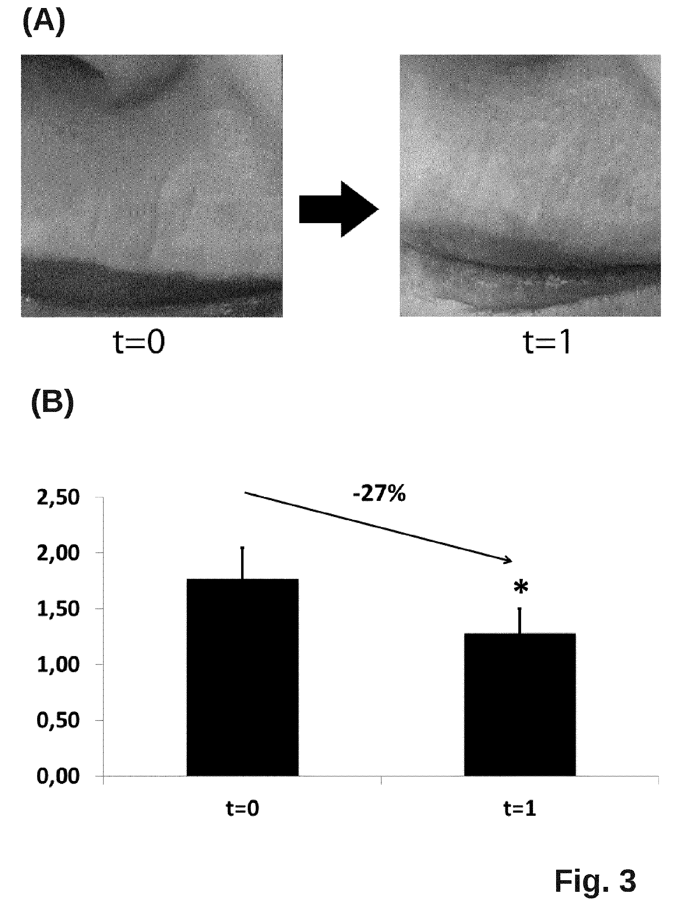

**Figur 3** **- Vergleich von Lippenfältchen vor der Behandlung und nach der Behandlung.** (A) Fotografierter Gesichtsbereich eines typischen Probanden bei Behandlungsbeginn (t=0) und nach der Behandlung (t=1). Das Ausmaß der Lippenfältchen n wurde durch die Behandlung mit Deferipron reduziert. (B) Median der Expertenbewertung der Lippenfältchen bei Behandlungsbeginn (t=0) und nach Ende der Behandlung (t=1) anhand einer fünfstufigen Skala von 1-5. Es zeigte sich eine statistisch signifikante Verbesserung durch die Behandlung mit Deferipron. n=20 (20 Probanden, die von 7 Experten bewertet wurden), die kleinen Balken repräsentieren die jeweilige Standardabweichung.

### Beispiel 1 - Wirksamkeitsstudie

Im Rahmen einer 4-wöchigen Wirksamkeitsstudie wendeten 20 Probanden im Alter zwischen 45 und 65 Jahren während der Dauer der Studie täglich eine kosmetische Creme Öl-in-Wasser-Emulsion (O/W-Emulsion, Rezeptur siehe folgenden Absatz), auf Basis der Basiscreme DAC, einer Stammzubereitung des Deutschen Arzneimittel-Codex (DAC), zusätzlich enthaltend 5% (w/w) Deferipron, Vitamin A und einen Lichtschutzfaktor 30, auf der Haut, insbesondere im Gesicht, an.

Formulierung Hautemulsion O/W (Konzentrationsangaben sind w/w):
3,7% Glycerolmonostearat 60
5,6% Cetylalkohol
6,9% Mittelkettige Triglyceride
23,5% Weißes Vaselin
6,5% Macrogol-20-glycerolmonostearat
9,3% Propylenglycol
37% gereinigtes Wasser
5,0% (w/w) Deferipron
0,5% Vitamin A
2,0% Bis-Ethylhexyloxyphenol Methoxyphenyl Triazine (BEMT)

Bei den Probanden waren zum Zeitpunkt des Beginns der Studie Zeichen von Hautalterung im Gesichtsbereich geringer bis mittlerer Ausprägung zu bemerken. Insbesondere feine Fältchen, Falten und verringerte Straffheit (Festigkeit, "firmness") und Elastizität.

### A. EXPERTENBEWERTUNG

Um die Wirksamkeit (Effektivität) von Deferipron in der erfindungsgemäßen Zusammensetzung im Sinne einer Reduzierung der Anzeichen der Hautalterung zu messen, wurden frontale und laterale Fotoaufnahmen des Gesichts, bei identer Ausleuchtung, vorgenommen, zum Zeitpunkt t=0 (Studienanfang/Kontrollgruppe) und zum Zeitpunkt t=1 (Ende der Studie).

Die Auswertung der Ergebnisse erfolgte anschließend nach einer in der Literatur bekannten, validierten Bewertungsmethodik für die objektive Messung von Zeichen und Ausmaß der Hautalterung. Siehe die Veröffentlichungen: R. NARINS et al., Validated Assessment Scales for the Lower Face, Dermatol Surg 2012;38:333-342. J. CARRUTHERS et al., Validated Assessment Scales for the Mid Face, Dermatol Surg 2012;38:320-332. T. FLYNN et al., Validated Assessment Scales for the Upper Face, Dermatol Surg 2012;38:309-319.

Als aussagekräftige Zeichen für die Bewertung der Hautalterung wurden folgende 7 Parameter definiert:
1. Feine Fältchen
2. Nasolabialfalten
3. Marionettenfalten
4. Lippenfältchen
5. Krähenfüße
6. Glabellafalten
7. Stirnfalten

Jeder der 7 Parameter wurde anhand der zum Zeitpunkt 0 (Studienanfang) und zum Ende der Studie angefertigten Fotoaufnahmen unter Verwendung der 5-stufigen Bewertungsskala bewertet (siehe z.B. Fig. 1 von Flynn et al.). Die Stufen der Bewertungsskale reichen von 0 (keine Zeichen) zu 4 (sehr starke und sichtbare Zeichen). Die Bewertung der Bilder wurde durch 7 Personen mit medizinischer Ausbildung vorgenommen.

Für die statistische Auswertung des Behandlungserfolges wurde für jeden Parameter einzeln, jeweils zu Beginn und Ende der Studie, der Median aller Fotobewertungen gebildet. Danach wurden die Mediane miteinander verglichen.

### B. SELBSTAUSKUNFT DER PROBANDEN

Weiters wurde nach Ende der Studie ein Fragebogen zur Selbstevaluierung an die Probanden verteilt und die Ergebnisse ebenfalls ausgewertet. Folgende Fragen wurden gestellt:
1. Bewerten Sie die Verbesserung der Festigkeit der Haut.
2. Bewerten Sie die Verbesserung der Hautelastizität.
3. Bewerten Sie die Reduzierung der Faltentiefe.
4. Bewerten Sie die feuchtigkeitsspendende Wirkung.
5. Würden Sie die Behandlung fortsetzen?
6. Würden Sie die Behandlung weiterempfehlen?

Die Beurteilung der Zufriedenheit mit den Ergebnissen erfolgte mit den Noten 1-4 (1= sehr zufrieden, 2= zufrieden , 3= etwas zufrieden, 4= nicht zufrieden).

### C. ERGEBNISSE

Für jeden der ausgewählten Parameter ließ sich anhand der vorgestellten Bewertungsmethodik, trotz der vergleichsweise kurzen Behandlungsdauer, bereits eine deutliche Verbesserung, ausgedrückt durch einen abnehmenden Median der sichtbaren Zeichen der Hautalterung, beobachten (siehe Tabelle 1). Siehe auch Figuren 1-3.

**Tabelle 1: Median der Expertenbewertung von sieben Hautalterungsparametern bei Behandlungsbeginn (t=0) und nach Ende der Behandlung (t=1), n=20 (20 Probanden, die von 7 Experten bewertet wurden).**

| | t=0 | t=1 | Verbesserung in % |
|---|---|---|---|
| Feine Fältchen | 2,94 | 2,26 | 23% |
| Nasolabialfalten | 3,14 | 2,61 | 17% |
| Marionettenfalten | 2,13 | 1,73 | 19% |
| Lippenfältchen | 1,76 | 1,28 | 27% |
| Krähenfüße | 2,74 | 2,14 | 22% |
| Glabellafalten | 2,18 | 1,79 | 18% |
| Stirnfalten | 2,63 | 2,21 | 16% |

Bei allen evaluierten Parametern lässt sich ein klar positiver Trend feststellen. Für die Parameter "Feine Fältchen", "Lippenfältchen" und "Krähenfüße" konnten trotz der begrenzten Probandenanzahl bereits statistisch signifikante Verbesserungen festgestellt werden. Für die Bestimmung der Signifikanz der Studienergebnisse wurde aufgrund der zwei nicht-normalverteilten verbundenen Stichproben der Wilcoxon-Test durchgeführt, wobei eine statistische Signifikanz dann als gegeben angenommen wurde, wenn der p-Wert kleiner/gleich 0,05 ist.

Auch die Auswertung des Fragebogens (vgl. Tabelle 2) zeigt, dass die Probanden mit den Ergebnissen mehrheitlich sehr zufrieden bis zufrieden waren. 9 von 10 Probanden würden die Behandlung fortsetzen.

**Tabelle 2: Selbstauskunft der Probanden, n=20. Die Bewertung erfolgte nach mit einem Schulnotensystem mit den Noten 1-4 (1= sehr zufrieden, 2= zufrieden, 3= etwas zufrieden, 4= nicht zufrieden).**

| | Bewertung (1-4) | "Ja" in % |
|---|---|---|
| Verbesserung der Festigkeit der Haut. | 1,4 | |
| Verbesserung der Hautelastizität. | 1,3 | |
| Reduzierung der Faltentiefe. | 1,5 | |
| Feuchtigkeitsspendende Wirkung. | 1,8 | |
| Würden Sie die Behandlung fortsetzen? | | 90% |
| Würden Sie die Behandlung weiterempfehlen? | | 83% |

Die Ergebnisse zeigen klar, dass Deferipron ein effektiver Wirkstoff für Zwecke der vorliegenden Erfindung ist.

Auch die nachfolgenden Beispiele dienen der beispielhaften Beschreibung der gegenständlichen Erfindung. Die Erfindung ist nicht auf die angeführten Ausführungsformen beschränkt. Alle Mengenangaben, Prozentangaben oder Teile beziehen sich, soweit nicht anders angegeben, auf das Gewicht, insbesondere das Gesamtgewicht der Zubereitungen oder der jeweiligen Mischung.

### Beispiel 2 Hautcreme W/O

0,6% Ubichinon-10
4,0% Deferipron
2,5% Cetearyl Alcohol / PEG-40 Castor Oil / Sodium Cetearyl Sulfate
6,0% Glycerin DAB 9
14,0% Vaseline DAB 9
43,0% Paraffinöl DAB 9
ad 100% gereinigtes Wasser

### Beispiel 3 Hautlotion W/O

2,0% All-trans-retinol
6,0% Deferipron
0,5% Aluminiumstearat
1,2% Phytoen
2,0% Glucosesesquiisostearat
4,0% Petrolatum
5,0% Glycerin DAB 9
19,0% Paraffinöl DAB 9
ad 100% gereinigtes Wasser

### Beispiel 4 Hautlotion O/W

0,4% Ubichinon-10
3,5% Deferipron
4,8% Glycerin DAB 9
5,5% Octyldodecanol
7,5% Cetearylisononanoat
9,0% Cetearyl Alcohol / PEG-40 Castor Oil / Sodium Cetearyl Sulfate
10,0% Paraffinöl DAB 9
ad 100% gereinigtes Wasser

## Patentansprüche

1. Verwendung von Deferipron zur kosmetischen Behandlung einer gesunden Hautstelle eines Individuums, umfassend die Anwendung einer Deferipron enthaltenden Zusammensetzung auf die Hautstelle.

2. Verwendung gemäß Anspruch 1, wobei die Hautstelle aus gealterter Haut, insbesondere chronologisch gealterter Haut, besteht.

3. Verwendung gemäß Anspruch 1 oder 2, um die Elastizität und/oder Straffheit der Hautstelle zu erhöhen.

4. Verwendung gemäß einem der Ansprüche 1 bis 3, um die Dermis der Hautstelle, insbesondere das Stratum reticulare, zu behandeln.

5. Verwendung gemäß einem der Ansprüche 1 bis 4, um zumindest ein Zeichen von Hautalterung, vorzugsweise in der Dermis der Hautstelle, zu behandeln.

6. Verwendung gemäß Anspruch 5, wobei die Hautalterung die chronologische Hautalterung ist.

7. Verwendung gemäß Anspruch 5 oder 6, wobei das Zeichen der Hautalterung ausgewählt ist aus Falten, Fältchen, schrumpeliger Haut und schlaffer Haut.

8. Verwendung gemäß einem der vorhergehenden Ansprüche, wobei das Alter das Individuums zumindest 30 Jahre, bevorzugt zumindest 40 Jahre, mehr bevorzugt zumindest 50 Jahre, noch mehr bevorzugt zumindest 60 Jahre, insbesondere zumindest 70 Jahre oder gar 80 Jahre beträgt.

9. Verwendung gemäß einem der vorhergehenden Ansprüche, wobei sich die Hautstelle mit Ausnahme der unteren Augenpartien im Gesicht, am Hals, am Rumpf oder an den Gliedmaßen des Individuums befindet.

10. Zusammensetzung geeignet zur Verwendung gemäß einem der Ansprüche 1 bis 9, umfassend Deferipron in einer Konzentration (w/w) von 1%-10%, bevorzugt von 2%-8%, mehr bevorzugt von 3%-7%, noch mehr bevorzugt von 4%-6%, insbesondere von im Wesentlichen 5%.

11. Zusammensetzung gemäß Anspruch 10, ferner umfassend ein kosmetisch verträgliches Mittel zur Verstärkung der Hautpenetration von Deferipron.

12. Zusammensetzung gemäß Anspruch 10 oder 11, ferner umfassend Hyaluronsäure.

13. Zusammensetzung gemäß einem der Ansprüche 10 bis 12, ferner umfassend Vitamin A, insbesondere all-trans-Retinylpalmitat, und/oder Vitamin C.

14. Verwendung gemäß einem der Ansprüche 1 bis 9, wobei die Deferipron enthaltende Zusammensetzung die Zusammensetzung gemäß einem der Ansprüche 10 bis 13 ist.
